# EUROPEAN PATENT SPECIFICATION

(11) **EP 3 215 440 B1**
(45) Date of publication and mention of the grant of the patent: **05.01.2022**
(21) Application number: 15798608.4
(22) Date of filing: 06.11.2015
(51) Int. Cl.: B65D 75/32, B05B 11/04, B65D 75/36, B65D 81/24, B65D 55/02, B65B 55/02

(54) **MEDICAL DEVICE PACKAGING**
GEHÄUSE FÜR MEDIZINISCHE VORRICHTUNG
EMBALLAGE DE DISPOSITIF MÉDICAL

(30) Priority: 07.11.2014 US 201462077132 P
(43) Date of publication of application: 13.09.2017
(73) Proprietor: Corium, Inc., Grand Rapids MI 49512 (US)
(72) Inventor: FAASSE, Adrian, L., Grand Rapids, MI 49512 (US); SINGH, Parminder, Union City, CA 94587 (US)
(74) Representative: D Young & Co LLP
(86) International application number: PCT/US2015/059559
(87) International publication number: WO 2016/073908

(56) References cited:
- JP-A- 2006 271 781
- US-A- 4 660 721
- US-A- 5 868 244
- None

## Description

### TECHNICAL FIELD

The disclosure relates generally to device packaging that may be sterilized and sealed, and related features thereof. The package of the invention is defined in claim 1 or claim 2 .

### BACKGROUND

Packaging for devices, especially medical devices, are well known in the art. Packaging is varied and includes pouches formed of medical grade paper, films, fiber based materials, foils, among others. Many medical devices are produced in aseptic environments and/or require sterilization of the packaging. The ability to sterilize and maintain sterility of packaging is especially true for medical devices that are implanted in or otherwise contact the body to prevent infection. JP 2006-271781 describes a method for manufacturing a sterilized packaging bag. The method allows for steam sterilization of a medical product and provides increased ease of opening.

The MicroCor^{®} product is a biodegradable microstructure patch technology for transdermal delivery of agents. The MicroCor^{®} product must be produced in an aseptic environment and requires a batch drying process of approximately 12 hours. The packaging is hermetically sealed under nitrogen and is required to have a zero or near zero O₂ and MVTR transmission rate to maintain sterility of the device. Processing and packaging under aseptic conditions would require an enormous investment in aseptic processing and equipment as well as requiring a great amount of space.

There exists a current need for sterile packaging of devices and delivery systems. The need is especially seen for devices such as medical devices and delivery systems that require additional processing of the packaging once the device is placed in the packaging to meet regulatory standards and/or maintain the viability of the device. From JP 2006/271 781 and US 5 868 244 packages are known, which comprise the features of the preamble of claim 1.

### BRIEF SUMMARY

The following aspects and examples described and illustrated below are meant to be exemplary and illustrative, and are in no way intended to be limiting in scope.

A package according to the invention comprises the features defined in claim 1 or claim 2.

According to the invention, the first seal is positioned between the first planar member/section and the second planar member/section for sealing the first planar member/section to the second planar member/section. The first seal seals the first planar member/section and the second planar member/section at a periphery of either or both of the first planar member/section or the second planar member/section.

According to the invention, the second sealable member adheres the first and second planar members/sections together at a position between the at least one opening and the cavity such that the cavity is no longer in communication with the at least one opening.

According to the invention, the barrier is formed of an antimicrobial material. Further, the barrier is formed of a microbial impervious material. In further examples the antimicrobial material or microbial impervious material is comprised of polyolefin fibers. In yet more examples, the polyolefin is selected from polyethylene or polypropylene. In additional examples, the polyolefin fibers are nonwoven.

In examples, the at least one opening is formed in the first planar member or section and the barrier is attached to the first planar member or section such that the barrier covers an entirety of the at least one opening. In further examples, the at least one opening is formed in the second planar member or section and the barrier is attached to the second planar member or section such that the barrier covers an entirety of the at least one opening.

In examples, the barrier seal is positioned between the first planar member or section and the barrier to secure the barrier to the first planar member or section. In other examples, the barrier seal is positioned between the second planar member or section and the barrier to secure the barrier to the second planar member or section. In further examples, the barrier is a breathable barrier.

In examples at least one of the first planar member or section or the second planar member or section is formed of a material selected from a gas impermeable polymer and/or a metal foil. In further examples each of the first and second planar members or sections are formed of a metal foil. In additional examples, the metal foil is selected from an aluminum foil and a stainless steel foil.

In examples, at least one of the first seal, the second seal and/or the barrier seal is formed of an adhesive coating. In additional examples, the adhesive coating is selected from a heat sealable adhesive coating and a pressure sealable adhesive coating.

In examples, the package includes a desiccant. In some examples, the interior of the package is sterile when the first planar member or section and second planar member or section are sealed together. In further examples, the cavity has a shape selected from rectangular, square, polygonal, oval, or circular.

The first planar member or section overhangs at least a portion of the second planar member or section at an edge when the first and second planar members or sections are sealed together. In further embodiments, the first planar member or section and the second planar member or section each have an outer periphery where at least a portion of the outer peripheries are substantially aligned when the first and second planar members or sections are sealed together.

According to the invention, the second seal intersects the first seal at two sides of the periphery of the first and second planar members or sections. In other examples, the at least one opening includes at least two openings. In further examples, the at least two openings are covered by a single barrier. In other examples, each of the at least two openings are covered by a separate barrier.

In another aspect, a method of preparing an aseptic package containing a medical device or delivery system comprising the features of claim 16 is disclosed. The method generally comprises placing the medical device or delivery system in a cavity of a package as described herein; sealing the first substantially planar member or section to the second substantially planar member or section at the first seal; hermetically sealing the sealed package; and sealing the first planar member or section to the second planar member or section at the second seal such that the cavity is no longer in communication with the at least one opening. In examples, the method further comprises removing the portion of the sealed first and second planar members or sections containing the at least one opening. In further examples, hermetically sealing includes a process selected from at least one of vacuum drying, nitrogen purging, heat drying, and terminal sterilization. In other examples, hermetically sealing includes a process selected from a combination of at least two of the processes selected from vacuum drying, nitrogen purging, heat drying, and terminal sterilization.

In examples, the method comprises sterilizing the package after the first sealing step. In some examples, the sterilizing step is selected from at least one of ethylene oxide sterilization, gamma electron-beam sterilization, and/or low temperature oxidative sterilization. In further examples, the sterilizing step includes a combination of two or more of ethylene oxide sterilization, gamma electron-beam sterilization, and low temperature oxidative sterilization.

Additional examples of the present packaging, methods, and the like, will be apparent from the following description, drawings, examples, and claims. As can be appreciated from the foregoing and following description, each and every feature described herein, and each and every combination of two or more of such features, is included within the scope of the present disclosure provided that the features included in such a combination are not mutually inconsistent. In addition, any feature or combination of features may be specifically excluded from any example of the present invention.

Additional aspects and advantages of the present invention are set forth in the following description and claims, particularly when considered in conjunction with the accompanying examples and drawings.

### BRIEF DESCRIPTION OF DRAWINGS

FIGS. 1A-1B are illustrations of exemplary packaging showing a top view (FIG. 1A) and side view (FIG. 1B).
FIG. 2 is an exploded view of the packaging shown in FIGS. 1A-1B.
FIG. 3 is an illustration of an exemplary method of forming the packaging.

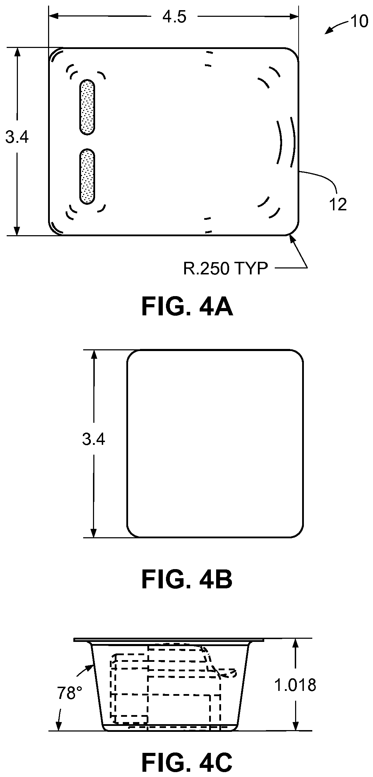

FIGS. 4A-4C are illustrations of exemplary packaging showing a top view with exemplary dimensions (FIG. 4A), a top view after trimming with exemplary dimensions (FIG. 4B); and a side view after trimming with exemplary dimensions (FIG. 4C).
FIGS. 5A-5B are illustrations of an exemplary packaging comprising a foldable or opposable planar substrate or member showing a top view (FIG. 5A) and a side view after folding or opposing the planar substrate or member (FIG. 5B).

### DETAILED DESCRIPTION

Various aspects of the packaging and related methods will be described more fully hereinafter. Such aspects may, however, be embodied in many different forms and should not be construed as limited to the examples set forth herein; rather, these examples are provided so that this disclosure will be thorough and complete, and will fully convey its scope to those skilled in the art.

The practice of the present disclosure will employ, unless otherwise indicated, conventional methods of mechanical engineering, chemistry, biochemistry, and pharmacology, within the skill of the art. Some of these techniques are explained fully in the literature. See, *e*.*g*.; A.L. Lehninger, Biochemistry (Worth Publishers, Inc., current addition); Morrison and Boyd, Organic Chemistry (Allyn and Bacon, Inc., current addition); J. March, Advanced Organic Chemistry (McGraw Hill, current addition); Remington: The Science and Practice of Pharmacy, A. Gennaro, Ed., 20th Ed.; Goodman & Gilman The Pharmacological Basis of Therapeutics, J. Griffith Hardman, L. L. Limbird, A. Gilman, 10th Ed.

Where a range of values is provided, it is intended that each intervening value between the upper and lower limit of that range and any other stated or intervening value in that stated range is encompassed within the disclosure. For example, if a range of 1 mm to 8 mm is stated, it is intended that 2 mm, 3 mm, 4 mm, 5 mm, 6 mm, and 7 mm are also explicitly disclosed, as well as the range of values greater than or equal to 1 mm and the range of values less than or equal to 8 mm.

### Definitions

As used in this specification, the singular forms "a," "an," and "the" include plural referents unless the context clearly dictates otherwise. Thus, for example, reference to a "polymer" includes a single polymer as well as two or more of the same or different polymers, reference to an "excipient" includes a single excipient as well as two or more of the same or different excipients, and the like.

In describing and claiming the present invention, the following terminology will be used in accordance with the definitions described below.

"Antimicrobial" as used herein refers to an agent or material that works against (e.g. kills or inhibits the growth of) microorganisms. "Antimicrobial" includes antibacterial, anti-mycobacterial, antifungal, antiviral, and anti-parasitic agents and materials.

"Biodegradable" refers to natural or synthetic materials that degrade enzymatically, non-enzymatically or both to produce biocompatible and/or toxicologically safe by-products which may be eliminated by normal metabolic pathways.

"Breathable" as used herein refers to the ability of a material to allow transmission of air, water and/or moisture vapor through the material. The breathability of a material may be determined in part by determining the moisture vapor transmission rate (MVTR) (also water vapor transmission rate (WVTR)) as a measure of the passage of gaseous water through a material or barrier. In one embodiment, MVTR is measured in g/m²/day using a measurement chamber and usually under known temperature and humidity conditions. The lower the MVTR rate, the longer the package prevents movement of moisture across the barrier or material.

"Gas impermeable" refers to a material or substance that is impermeable to (e.g. does not allow or substantially does not allow passage of) one or more gases across the material or substance.

"Hydrophobic polymer" as used herein refers to polymers that are insoluble or poorly soluble in aqueous solvents. "Hydrophilic polymer" as used herein refers to polymers that are soluble or substantially soluble in aqueous solvents.

"Medical device" as used herein refers to any of a number of devices relating to or used in medical procedures. Examples include, but are not limited to diagnostic products or devices, general purpose laboratory equipment, reagents and reagent containers, test kits, and treatment devices and kits.

"Microbe" refers to microscopic organisms. Microbes include, without limitation, bacteria, fungi, algae, animals, plants, and viruses.

"Microbial-resistant" refers to resistance by a material to entry and/or passage of microbes. "Microbial impervious" in relation to a material refers to a material that does not allow microbes or microbial agents to enter and/or pass through.

"Moisture-resistant" refers to resistance by a material to permeability of moisture or water.

"Optional" or "optionally" means that the subsequently described circumstance may or may not occur, so that the description includes instances where the circumstance occurs and instances where it does not.

"Oxygen Transmission Rate" (OTR or O₂) is a measurement of the amount of oxygen that passes through a substance or material in a given period of time.

"Substantially" or "essentially" means nearly totally or completely, for instance, 80-85%, 80-90%, 80-95%, 85-90%, 85-95%, 90-95% or greater of some given quantity.

"Transdermal" refers to the delivery of an agent into and/or through the skin for local and/or systemic therapy. The same principles apply to administration through other biological membranes such as those which line the interior of the mouth, gastro-intestinal tract, blood-brain barrier, or other body tissues or organs or biological membranes which are exposed or accessible during surgery or during procedures such as laparoscopy or endoscopy.

A material that is "water-soluble" may be defined as soluble or substantially soluble in aqueous solvents. A material that is "water-soluble" preferably dissolves into, within or below the skin or other membrane which is substantially aqueous in nature.

### Overview

The present disclosure is directed, at least in part, to systems, devices, and methods relating to sterile or aseptic packaging, especially packaging for medical devices and/or delivery systems.

### System and Device

Before describing the present subject matter in detail, it is to be understood that this invention is not limited to specific materials or device structures, as such may vary. It is also to be understood that the terminology used herein is for the purpose of describing particular examples only, and is not intended to be limiting.

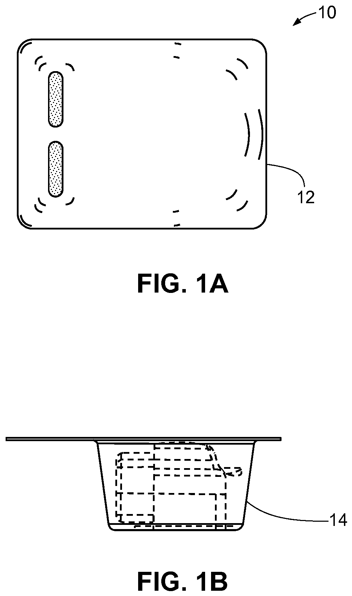

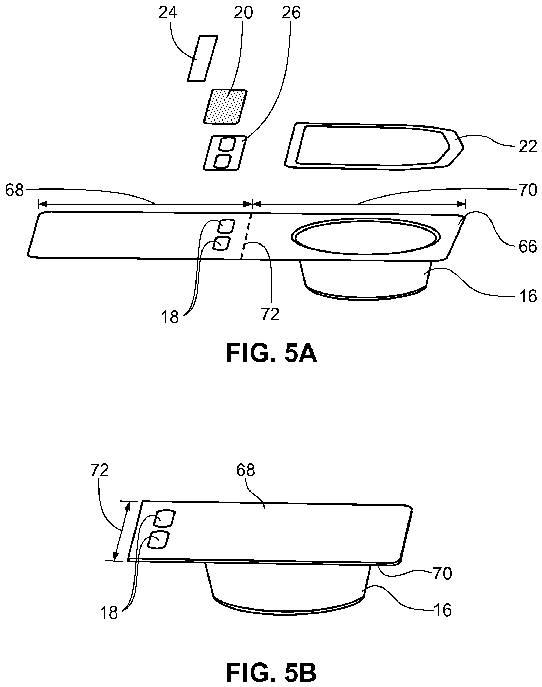

In one aspect, a sterilizable, sealable packaging for a medical device or delivery system is described herein. The packaging may be used to contain any apparatus, device, or system that should be maintained in a sterile, aseptic, or hermetically sealed environment. An embodiment of an exemplary packaging system is shown in Figs. 1A-1B. Fig. 1A is a top view looking down at the packaging system from an elevated position. Fig. 1B is a side view of the packaging system showing a device contained within the package. Fig. 1B shows a microprojection device and applicator as the medical device. The discussion of the figures and packaging below is with reference to the device shown in Fig. 1B and 5B. However, it will be appreciated that the packaging may be suitable for use with any device or system that requires sterilized, aseptic, or hermetically sealed packaging.

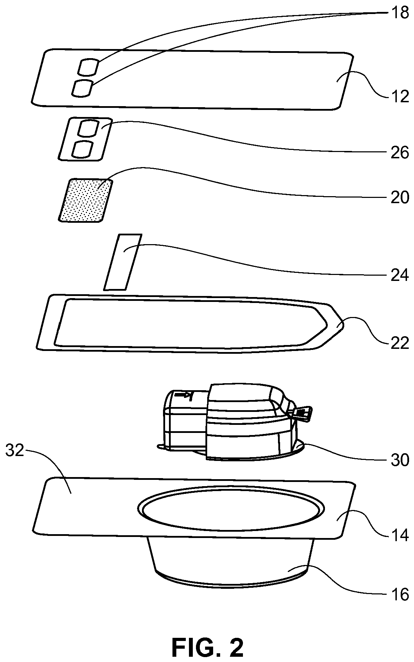

As seen in Figs. 1A-1B, the packaging system 10 includes a first substantially planar member 12 having first and second opposed sides and a second substantially planar member 14 having first and second opposed sides. In the embodiment shown in Fig. 1A, the first planar member 12 has a rectangular shape. It will be appreciated that the first planar member may have any suitable shape including, but not limited to, rectangular, square, circular, elliptical, and polygonal. The second member includes a cavity or depression 16 within a portion of the planar portion of the second planar member for holding the medical device 30. As seen in Fig. 2, the second substantially planar member includes a planar section or portion 32 and a cavity section or portion 16. In this embodiment, the cavity section or portion opening is surrounded by the planar section or portion. The cavity may be formed in the second planar member using any suitable means. In non-limiting embodiments, the cavity is formed by molding including injection molding and thermoforming. It will be appreciated that the cavity may be specifically molded to hold a particular device or apparatus. In another embodiment, a further holder, which may be a molded piece, may be included within the cavity to hold a particular device or apparatus. It will further be appreciated that both of the first planar member and the second planar member may include a cavity or depression. In one embodiment, when the first and second planar members are opposed, the cavities in the first and second members form a space suitable for containing the device.

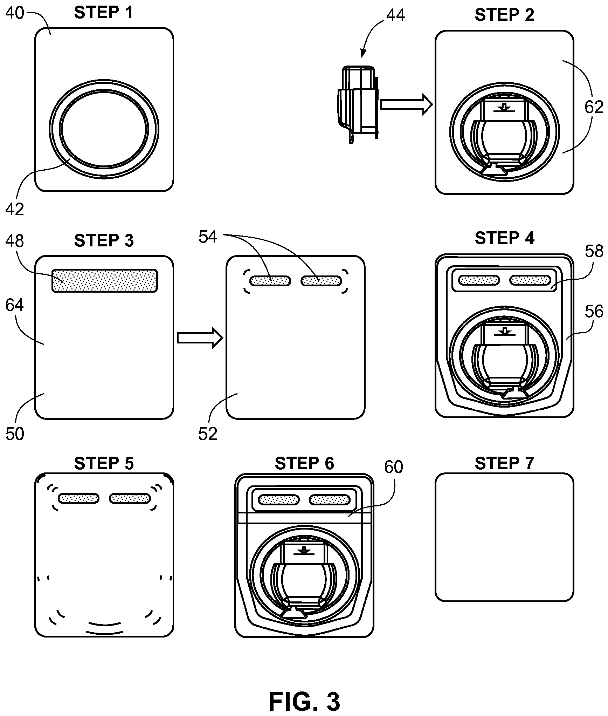

The first and second substantially planar members are opposable so that the first member covers at least a portion of the second member. Preferably, the second opposed side of the first planar member contacts and at least partially covers the first opposable side of the second planar member. In the embodiment shown in Figs. 1A-1B, the first member covers substantially all of the planar portion of the second member. It will be appreciated that the first member may cover only a portion of the planar portion of the second member. At least the cavity opening should be covered by the first planar member. It will be appreciated that at least a portion of the first and second substantially planar members have a surface that is conducive for contact with each other. For example, in the embodiment shown in Fig. 2, the first and second substantially planar members each have a flat portion where the members are contacted and sealed together. In an embodiment, the first and second substantially planar members each have an outer periphery, see items 62 and 64 in Fig. 3, where the outer peripheries are substantially aligned when the first and second substantially planar members are opposed or sealed.

The first and second substantially planar members may be formed or comprised of any material suitable for packaging a device and maintaining a sealed and/or sterile environment. Suitable materials are preferably gas and/or moisture impermeable. Suitable materials include, but are not limited to polymers, foils, and laminates. One suitable polymer is a gas and/or moisture impermeable polymer. In other embodiments, the polymer is a high barrier plastic that prevents moisture, oxygen, and/or other gases from permeating the packaging. In other embodiments, the polymer provides a barrier to ultraviolet light and/or radiation. Exemplary polymers include, but are not limited to, polyolefins, polyesters, acrylics and the like. In other embodiments, the polymers include, but are not limited to polyethylene teraphthalate (PET), polyethylene teraphtalate glycol (PETG), high-density polyethylene (HDPE), low-density polyethylene (LDPE), polyvinyl chloride (PVC), polyurethane, polypropylene (PP), polystyrene (PS), high impact polystyrene (HIPS), polycarbonate (PC), acrylonitrile butadiene styrene (ABS).

In one embodiment the foil is a metal foil. The metal foil may be comprised of any suitable metal that maintains sterility and does not react with the device contained therein. Exemplary metal foils include, but are not limited to, aluminum foil, stainless steel foil, and tin foil. In a further embodiment, the material used for the first and/or second planar member is a metalized polymer, a polymer/metal laminate, and/or a coated polymer. A polymer/metal laminate may be formed by any method as known in the art. In one non-limiting embodiment, the laminate comprises one or more layers of a suitable polymer that are coated onto a metal foil. In an embodiment, the metal foil laminate is a heat-sealable foil laminate. It will be appreciated that both the first and the second substantially planar members may be formed of a heat-sealable laminate foil. It will be appreciated that the first and second planar members may be formed or comprised of the same or different materials. Preferably, the material used for the first and/or second planar members can withstand, without limitation, steam, autoclaving, ethylene oxide, nitrogen, heat drying, vacuum drying, terminal sterilization via dry heat, chemical sterilization (including chlorine dioxide, vaporized hydrogen peroxide, and hydrogen peroxide plasma), and/or radiation (including gamma ray and electron beam) sterilization without being degraded or substantially degraded. The first and/or second planar member may further be coated with a material that reduces or decreases at least one of gas permeability, moisture permeability and ultraviolet light permeability.

As seen in Fig. 2, the second substantially planar member 14 includes at least one cavity 16 for holding or containing the device 30. The cavity should have a sufficient width, depth and shape to contain the device. The cavity can have any size or shape suitable for holding the device. Suitable shapes include circular, oval, elliptical, rectangular, square, polygonal, or combinations thereof. Preferably, at least a portion of the second substantially planar member around the cavity has a flat, planar, or otherwise regular surface. This surface allows for better sealing of the first substantially planar member to the second substantially planar member.

Although the Figs. 1 and 2 depict the first and second substantially planar members as separate, it will be appreciated that the members may be formed of a single planar material that is folded. As shown in Figs. 5A-5B, a substantially planar packaging member 66 includes a first portion 68 and a second portion 70. A cavity 16 as described above is formed at least one of the first or second portions. One or more openings 18 are further formed in at least one of the first and/or second portions. The planar packaging member is folded along an axis 72 such that the first and second portions are opposed when the planar member is folded (Fig. 5B). It will be appreciated that the further seal and barrier, etc. features and embodiments thereof described with reference at least to Figs. 1A-1B and 2 also apply to this embodiment. Briefly, a first seal 22 is positioned on at least one of the first or second portions for sealing the first section to the second section when the planar member is folded and the first and second sections are opposed. A microbial-resistant barrier 20 covers the at least one opening. A second seal 24 is positioned at a position between the at least one opening and the cavity. The second seal, when sealed, adheres the first and second portions together such that the cavity is no longer in communication with the at least one opening.

In one embodiment, the system includes a first sealing member or first seal for sealing at least a portion of the first substantially planar member to the second substantially planar member. In one embodiment, the first sealing member or first seal is formed from an adhesive coating on the first substantially planar member, the second substantially planar member, or both. It will be appreciated that the sealing member may be comprised of any material or structure that sufficiently adheres or attaches at least a portion of the first and second planar members. It will further be appreciated that the choice of adhesive may be guided by the materials used for the first and/or second planar members. In one embodiment, the sealing member is an adhesive seal positioned at a periphery of either or both of the first and second planar members. In a further embodiment, the sealing member is an adhesive seal positioned at least partially at a periphery of the cavity. As seen in Fig. 2, the first sealing member 22 may be a single sealing member positioned between the first 12 and second 14 planar members. It will be appreciated that the first sealing member may be a separate structure or may be an adhesive composition that is applied to the first and/or second planar members. Suitable adhesives include, but are not limited to, solvent-based, water-based, cold seal, and heat-seal or melt adhesives. In an embodiment, the adhesive is a pressure-sensitive adhesive or adhesive coating. In a further embodiment, the adhesive is a pressure sealable adhesive or adhesive coating. In another embodiment, the adhesive is a heat sealable adhesive or adhesive coating. In an embodiment, the first sealing member is a peelable adhesive such that the first planar member and second planar member may be separated to access the medical device within the cavity by peeling the first and/or second planar members apart. Exemplary adhesives include, but are not limited to acrylics, epoxies, silicones, cyanoacrylates, and styrene block co-polymers. Further exemplary adhesives include, but are not limited to, the Xhale^{®} adhesive with DotCoat^{®} technology and the SealScience^{®} water-based adhesive, both available from Oliver-Tolas^{®} (Grand Rapids, MI). Suitable pressure-sensitive adhesives include adhesive polymers or co-polymers. Polymers having auto-adhesion characteristics include, but are not limited to, natural rubber, polyisoprene, butyl, and certain formulations of silicone rubber. In an embodiment, the adhesive provides a hermetic seal. It will be appreciated that the adhesives may include appropriate excipients as known in the art. Once sealed, the adhesives used should maintain sterility of the interior of the packaging. Preferably, the adhesive is non-permeable at least to oxygen and/or moisture vapor. It will further be appreciated that, depending on the materials used for the first and/or second planar members, the members may be adhered or affixed together without the use of a sealing member. For example, the first and second planar members may be heat sealed by at least partially melting the members. In an embodiment, application of heat at a suitable selected temperature to the heat-sealable laminate causes the polymer to melt and fuse with or into the opposite planar member. If the first and second members are sealed without the use of an adhesive (e.g. by heat sealing), the seal should maintain sterility of the interior of the packaging. Preferably, the seal is non-permeable at least to oxygen and/or moisture vapor.

At least one of the first and/or second substantially planar members includes at least one opening or vent. The openings may be of any suitable size and shape to facilitate sterilization of the packaging and device. In the embodiment as shown in Figs. 1A and 2, two openings 18 are formed in the first substantially planar member. In other embodiments, at least one, two, three, four or five openings are formed in at least one of the first or second substantially planar members. In the embodiment shown in Fig. 1A, the openings are shown side by side. It will be appreciated that where multiple openings are included, the openings may be adjacent and/or set apart from each other. The openings or vents may be positioned in any suitable portion of the first and/or second planar members. However, the openings or vents should not be positioned over or within the cavity so that the openings may be sealed off from the cavity after sterilization or other processing.

The openings or vents are covered with one or more barrier materials. The barrier material is preferably a microbial-resistant barrier. In other embodiments, the barrier is microbial impervious. The barrier material allows for sterilization of the packaging and/or device contained therein. The barrier may be any suitable size or shape that allows for coverage of the opening or vent 18 in the first and/or second planar members. As seen in Fig. 2, a single barrier 20 may be used to cover multiple openings or vents 18. It will be appreciated that a separate barrier may be used to cover each separate opening or vent. It will be appreciated that more than one material may be used where multiple openings or vents are formed in the first and/or second planar members. Further, more than one material may be used to cover the openings or vents. For example, the barrier materials may be stacked, laminated, or positioned in overlying configurations. In another embodiment, the multiple openings/vents may be covered with different barrier materials. This may be advantageous where more than one sterilization or processing steps are performed. For example, different openings/vents may be covered with a barrier material suitable for use with the different sterilization or other processing steps. As an illustration, one of opening/vent may be covered with a barrier material that is permeable to steam and another covered with a material that is permeable to ethylene oxide gas. Regardless of the barrier material configuration, all of each of the openings or vents should be covered by at least one barrier material.

The barrier may be made of any suitable material that allows for and/or facilitates one or more sterilization procedures. In one embodiment, the barrier is a breathable material that is suitably permeable to permit sterilizing gases such as steam, ethylene oxide (EtO), hydrogen peroxide (H₂O₂), chlorine dioxide (CD), ozone, glutaraldehyde, peracetic acid, nitrogen, and/or Freon to pass the barrier. The barrier may additionally be transparent to allow for irradiative sterilization. The barrier is preferably impermeable or substantially impermeable to moisture, bacteria, fungi, viruses, and/or other substances that may compromise the sterility of the medical device and the packaging. In one embodiment, the barrier is formed of a microbial-resistant, microbial-impermeable, or microbial impervious material. One exemplary material is comprised of polyolefin including, but not limited to, one or more polyolefin fibers. In embodiments, the polyolefin fibers may be woven or non-woven. Polyolefins include, but are not limited to polystyrene, polycarbonate, acrylics, polyethylene, polypropylene, silicone rubber, and synthetic rubbers. In some embodiments, the material is comprised of polyethylene and/or polypropylene fibers. One suitable material for the barrier is the water resistive and antimicrobial barrier material sold under the trademark TYVEK^{®} available from DuPont (Wilmington, DE). TYVEK^{®} is a fabric made from spun high-density polyethylene (HDPE). Each of the Type 10, 14 or 16 styles of TYVEK^{®} may be suitable for use as the barrier material. Type 10 style products are hard or stiff products. Types 14 and 16 are fabric-like flexible products. The Type 14 style products are particularly suitable for use as the barrier material as they are flexible yet have lower moisture permeability than the Type 16 style products. Further exemplary materials for use as the barrier material include the P3 technology^{™} available from Porex^{®} (Fairburn, GA) and OVANTEX^{®} available from Oliver-Tolas Healthcare Packaging (Grand Rapids, MI). The P3 technology^{™} is a porous polytetrafluoroethylene (PTFE) material that provides venting capability for EtO sterilization. OVANTEX^{®} is an adhesive-coated medical grade material comprised of a blend of synthetic fibers and cellulose-based components. OVANTEX^{®} may be used with EtO and radiation (gamma irradiation) sterilization.

Optionally, a barrier seal is positioned at least partially between the barrier material and the first and/or second planar member to adhere or affix the barrier material. In one embodiment, the barrier seal is formed of an adhesive coating applied to the barrier material and/or the planar member to which the barrier material is attached. The barrier seal may be comprised of any material or structure that sufficiently adheres or attaches at least a portion of the first and second planar members to the barrier material. It will be appreciated that the selection of the barrier seal may depend upon the choice of barrier material and/or material for the first and/or second planar members to ensure the desired adhesion or affixation. The barrier seal may be formed of any adhesive as described above for the first sealing member. In an embodiment as shown in Fig. 2, the barrier seal 26 is positioned around a perimeter of the opening/vent 18 and/or the barrier material 20. It will be appreciated that the barrier seal may be positioned around a perimeter of the barrier material and contacting the first or second substantially planar member. Alternatively, the barrier member may be adhered or affixed to the first or second substantially planar member without the use of an adhesive.

The system includes one or more second sealing members or seals for sealing at least a portion of the first substantially planar member to the second substantially planar member. As seen in Fig. 2, the second sealing member 24 is positioned between the opening/vent 18 and the cavity 16. Prior to sterilization processes, the second sealing member is adhered or affixed to one of the first or second substantially planar members. After the sterilization procedure(s), the second sealing member is adhered or affixed to the other of the first or second substantially planar members. In this manner, the cavity may be sealed apart from the openings/vents to preserve the sterility of the cavity and the contents therein. The second sealing member may be placed in any suitable position such that the opening/vent is sealed from contact with the cavity. In the embodiment shown in Fig. 2, the second seal 24 is placed across the first and second substantially planar members with the ends being in contact with the first sealing member. In some embodiments, the second sealing member intersects the first sealing member at two sides of the periphery of the first and second substantially planar members. Once the second sealing member is sealed, the cavity is sealed apart from the opening/vent by the first and second sealing members. In other embodiments, the second sealing member may be placed around a perimeter of the openings/vents. In embodiments where more than one opening/vent is included at positions apart from each other, a separate second or secondary sealing member may be used to seal off multiple or each of the opening/vents. It will also be appreciated that where the packaging includes two, or more, openings/vents that are spaced apart, multiple secondary seals may be positioned between the multiple openings/vents and the cavity. The secondary seals may be used to seal contact between all of the multiple openings/vents or only a portion of the multiple openings/vents at one time. For example, the packaging may include a first vent that is covered with a barrier material that is steam permeable and a second vent that is covered with a barrier material that is permeable to ethylene oxide. The packaging may first be autoclaved and then ethylene oxide sterilized. The secondary seal associated with the steam permeable barrier material may be sealed apart from the cavity prior to ethylene oxide sterilization. In this manner, the packaging may be sequentially sterilized with openings associated with each sterilization step being sealed after the appropriate sterilization step. The multiple secondary seals may be formed of the same or different materials. In an embodiment, the second sealing member or seal is formed from an adhesive coating on the first substantially planar member and/or the second substantially planar member.

The second sealing member may be may be comprised of any material or structure that sufficiently adheres or attaches at least a portion of the first and second planar members together. It will be appreciated that the selection of the second sealing member may depend upon the choice of material for the first and/or second planar members to ensure the desired adhesion or affixation. The second sealing member may further be formed of any adhesive as described above for the first sealing member. The second sealing member may be any suitable shape for sealing the opening/vent from contact with the cavity. In the embodiment shown in Fig. 2, the second sealing member 24 is rectangular and extends transverse across the first and second substantially planar members 12, 14. In other embodiments, the second sealing member may be positioned around the opening/vent. The second sealing member may be, without limitation, rectangular, square, circular, or elliptical.

The packaging, when assembled, may include any feature or configuration that improves ease of opening the sealed package. In an embodiment, one of the first or second substantially planar members overhangs at least a portion of the opposing member. In some embodiments, the overhang makes it easier for a user to grip the overhanging member and separate the first and separate members to access the device. In another embodiment, one of the first or second members includes a tab or otherwise stiffer portion to ease grip and/or separation of the first and second members. The overhang may be at a corner, at one side or at a perimeter of the first or second members.

Packaging may further include any suitable feature for maintaining the contents in a suitable and sterile or aseptic environment. In one embodiment, the packaging includes one or more desiccants. In an embodiment, the packaging includes a desiccant at least in the cavity. Any suitable desiccant as known in the art is suitable for use with the packaging.

It will be appreciated that the embodiments and features described with respect to specific elements above may be combined with the embodiments and features described with respect to other specific elements. For example, and without limitation, the specific embodiments of the planar member(s), seals, barrier, etc. may be combined in the contemplated packaging.

### Methods of Use

The methods, kits, packaging, and related devices described herein are used to provide sterile packaging for an apparatus, medical device, or delivery system. The packaging is configured such that the packaged device may be sterilized within the packaging, which can thereafter be easily sealed. In one aspect, a method of preparing an aseptic or sterilized package containing a device such as a medical device, apparatus or delivery system is contemplated.

An exemplary process of packaging a medical device, or other device or apparatus, is shown in Fig. 3. The packaging process is described hereafter with reference to Fig. 3, which shows one particular embodiment of the packaging system in use. However, it will be appreciated that other embodiments of the system as described above are suitable for use with the methods described below.

The packaging method is described below with reference to some particular steps. It will be appreciated that further steps may be included with the described method. It will further be appreciated that some steps may be omitted or combined as appropriate. In a first step, a first member 46 and a second member 40 are obtained or manufactured. At least the second member 40 is formed with a cavity or depression 42 in a substantially planar member. The cavity or depression may have any suitable shape or size to contain the device. In some embodiments, the second member is formed of a moldable or shapeable metal or polymer. In the embodiment as shown in Fig. 3, the second member 40 is a pre-formed aluminum shell. The first member 46 has first 52 and second 50 opposing sides. The first member may be formed of any suitable material as described above. The first and/or second member is formed with one or more vents or openings 54 extending through the first and/or second member. In the embodiment shown in Fig. 3, two vents are formed in the first member. The vents or openings are covered with one or more barrier materials 48. The barrier material is typically, but not always, affixed to the second side 50 of the first member. As also seen in Fig. 3, one or more barrier seals 58 are included to adhere or affix the barrier material 48 over the vents/openings and to the first member 46. In this embodiment, the barrier seal 58 is adhered to the barrier material 48 around a perimeter of and overlapping the barrier material 48. In this embodiment, the barrier seal contacts both a perimeter of the barrier material and the second side 50 of the first member 46. It will be appreciated that the barrier 48 may be affixed or adhered to the first member 46 by any suitable means. In embodiments, the barrier material is affixed or adhered to the first member without the use of a separate barrier seal. In the embodiment of Fig. 3, a rectangular barrier material formed of Tyvek^{®} material is sealed, affixed, or adhered to the second side of the first member. In this embodiment, a single barrier material is used to cover both of the vents formed in the first member.

In a second step, the device 44 is placed within the cavity or depression 42 in any suitable manner. In a third step, the first member 46 is placed at least partially over the second member 40. The first member is placed so that at least the cavity 42 is covered by the first member 46. In the embodiment as shown in Fig. 3, the second side 50 is placed adjacent the second member 40. The first member 46 functions as a cover or lid for the cavity. In a fourth step, the first member 46 is affixed or adhered to the second member 40 using any suitable seal, adhesive or other mechanism. In the embodiment as shown in Fig. 3, a perimeter heat seal 56 is included between the first and second members. Once the first member is placed over the second member, the packaging is sealed. In an embodiment, the packaging is heated, at least at the perimeter seal, to a suitable temperature to adhere the first and second members together. It will be appreciated that the first and second members should be sealed such that a perimeter at least around the barrier material and the cavity are sealed. Preferably, the seal is a hermetic seal to preserve the sterility of the packaging after sterilization. In a fifth step, the packaging is subjected to one or more suitable sterilization processes or procedures. In an embodiment, at least one of the sterilization processes makes use of the barrier material and the vent. For example, in one embodiment, the sterilization process is gas sterilization where the barrier material is permeable to the gas used in the sterilization process. In another embodiment, the sterilization process uses a vacuum. Any suitable sterilization process or combination or sterilization processes may be used. In some embodiments, the packaging is sterilized by one or more of autoclaving, gas purging, vacuum drying, heat drying, irradiation, low temperature oxidative sterilization, and/or terminal sterilization via dry heat. In other embodiments, the packaging is sterilized by irradiation. In an embodiment the irradiation comprises gamma and/or electron-beam sterilization. In some embodiments, the gas purging uses steam, EtO, H₂O₂, chlorine dioxide, nitrogen, peracetic acid, and/or Freon as the gas. In embodiments, the packaging is sterilized by one or more processes described herein or known to those of skill in the art to be suitable for sterilizing packaging. In another embodiment, the packaging is sterilized by steam autoclaving and/or dry autoclaving. In other embodiments, the packaging is sterilized using gas plasma technology. It will be appreciated that the packaging may be sterilized using at least two or two or more of the above recited processes. In one exemplary combination, the packaging is sterilized by two or more processes selected from gas purging, irradiation, and low temperature oxidative sterilization. In another exemplary combination, the packaging is sterilized by two or more processes selected from EtO sterilization, gamma irradiation, electron-beam sterilization, and low temperature oxidative sterilization. The FDA (fda.gov) lists several traditional methods for sterilization of medical devices including dry heat sterilization, moist heat sterilization, EtO sterilization using devices placed in a fixed chamber, radiation (gamma and electron-beam), and liquid chemical sterilants. The FDA also lists several other methods of sterilizing medical devices including EtO not using a fixed chamber (e.g. porous polymer bag, diffusion method, sterilization pouch, injection, etc.), high intensity light, chlorine dioxide, ultraviolet light, combined vapor and gas plasma, and vapor systems (e.g. peroxide or peracetic acid). In embodiments, the packaging is sterilized using one or more of the methods described by the FDA. A sterility assurance level (SAL) of 10⁻³ is generally accepted as adequately sterile for many medical devices. In other embodiments, the packaging is sterilized to achieve a SAL of between at least about 10⁻³ to about 10⁻⁶. In specific embodiments, the packaging is sterilized to achieve a SAL of at least about 10⁻³, about 10⁻⁴, about 10⁻⁵, or about 10⁻⁶. In further embodiments, the packaging is sealed to maintain a SAL of between at least about 10⁻³ to about 10⁻⁶. In specific embodiments, the packaging is sealed to maintain a SAL of at least about 10⁻³, about 10⁻⁴, about 10⁻⁵, or about 10⁻⁶.

In a sixth step, a portion of the first and second members are sealed between the openings/vents and the cavity. In an embodiment, a second seal 60 is positioned between the vents/openings and the cavity to seal the first and second members. Placement of the second seal is not critical; however, the second seal should be positioned such that the vents/openings are sealed apart from the cavity when the second seal is adhered to the first and second members. In the embodiment as shown in Fig. 3, the second seal 60 is a rectangular cross seal that is positioned transverse across the first and second members and contacts the perimeter seal 56 at both ends. It will be appreciated that the second seal is not initially sealed to both of the first 46 and the second members 40. Accordingly, the openings/vents are initially in communication with the cavity such that the packaging interior may be sterilized through the vents/openings. Optionally, in a seventh step, at least a portion of the first and/or second members is cut or otherwise removed to present a more compact packaging. In the embodiment as shown in Fig. 3, the vent section is removed, this does not form part of this invention. Where a portion of the packaging is removed, the cavity should remain sealed at a perimeter to preserve the sterility of the packaging interior.

Alternatively, a planar member having a first portion and a second portion is obtained or manufactured. One or both of the first and second portions includes a cavity. The device is placed in the cavity as described above. The planar member is folded such that at least a portion of the first and second portions are opposed and the cavity is covered by the other of the first or second portions. The planar member is then sealed and processed as described above.

It will be appreciated that the embodiments and features described with respect to specific steps of the method may be combined with the embodiments and features described with respect to other specific steps of the method.

While a number of exemplary aspects and embodiments have been discussed above, those of skill in the art will recognize certain modifications, permutations, additions and subcombinations thereof.

## Claims

1. A package (10) for a medical device (30) or delivery system, comprising:
a first member (12), wherein the first member is substantially planar;
a second member (14) comprising at least one cavity (16) for receiving a medical device (30) or delivery system, wherein the first and second members are opposed such that the first member covers at least the cavity of the second planar member;
a peripheral seal (22) positioned between the first member and the second member for sealing the first member to the second member at the periphery of either or both of the first member or the second member;
at least one opening (18) formed in at least one of the first or second members;
a microbial-resistant barrier (20) covering the at least one opening (18); **characterized by** a sealable member (24) positioned between the first member and the second member, that adheres the first and second members together at a position between the at least one opening and the cavity such that when the first and second members are adhered by the sealable member the cavity is no longer in communication with the at least one opening, wherein the at least one opening (18) covered by the microbial-resistant barrier (20) is present in the package (10)after the cavity is no longer in communication with the at least one opening.

2. A package (10) for a medical device (30) or delivery system, comprising:
a member (66) having a first section (68) that is substantially planar and a second section (70) adjacent the first section;
at least one cavity (16) for receiving medical device or delivery system positioned in the second section, wherein the member (66) is foldable along an axis (72) between the first and second sections such that the first and second sections are opposed and the first section covers at least the cavity of the second section when the sections are opposed;
a peripheral seal (22) positioned on at least one of the first or second sections for sealing the first section to the second section at the periphery of either or both of the first section or the second section when the sections are opposed;
at least one opening (18) formed in at least one of the first or second sections;
a microbial-resistant barrier (20) covering the at least one opening; and
a sealable member (24) that adheres the first and second sections together at a position between the at least one opening and the cavity such that when the first and second sections are adhered by the sealable member the cavity is no longer in communication with the at least one opening, wherein the at least one opening (10) covered by the microbial-resistant barrier (20) is present in the package (10) after the cavity is no longer in communication with the at least one opening.

3. The package of claim 1 or 2, wherein the barrier (20) is formed of an antimicrobial material or a microbial impervious material,
preferably wherein the antimicrobial material or microbial impervious material is comprised of polyolefin fibers, and
more preferably wherein the polyolefin is selected from polyethylene or polypropylene, and wherein the polyolefin fibers are nonwoven.

4. The package of any previous claim, wherein the at least one opening (18) is formed in the first member (12) or first section (68) and the barrier (20) is attached to the first member or the first section covering the entirety of the at least one opening, or
wherein the at least one opening (18) is formed in the second member (14) or second section (70) and the barrier is attached to the second member or second section covering the entirety of the at least one opening.

5. The package of claim 4, further comprising a barrier seal (26) positioned between the first member (12) or first section (68) and the barrier to secure the barrier to the first member or first section, or a barrier seal (26) positioned between the second member (14) or second section (70) and the barrier to secure the barrier to the second member or second section.

6. The package of any previous claim, wherein the barrier (20) is a breathable barrier.

7. The package of any previous claim, wherein at least one of the first member (12) or first section (68) or the second member (14) or second section (70) is formed of a material selected from a gas impermeable polymer and a metal foil,
preferably wherein each of the first and second members or sections are formed of a metal foil, and more preferably wherein the metal foil is selected from an aluminum foil and a stainless steel foil.

8. The package of any previous claim, wherein at least one of the peripheral seal (22), the sealable member (24) or the barrier seal (26) is formed of an adhesive coating,
preferably wherein the adhesive coating is selected from a heat sealable adhesive coating and a pressure sealable adhesive coating.

9. The package of any previous claim, further including a desiccant.

10. The package of any previous claim, wherein the interior of the package is sterile when the first member (12) or first section (68) and second member (14) or second section (70) are sealed together.

11. The package of any previous claim, wherein the cavity (16) has a shape selected from rectangular, square, polygonal, oval, or circular.

12. The package of any previous claim, wherein the first member (12) or first section (68) overhangs at least a portion of the second member (14) or second section (70) at an edge when the first and second members or sections are sealed together.

13. The package of any previous claim, wherein the first member (12) or first section (68) and the second member (14) or second section (70) each have an outer periphery where the outer peripheries are substantially aligned when the first and second members or sections are sealed together.

14. The package of any previous claim, wherein the sealable member (24) intersects the peripheral seal (22) at two sides of the periphery of the first and second members or sections.

15. The package of any previous claim, wherein the at least one opening (18) includes at least two openings, wherein each of the at least two openings are covered by a single barrier or wherein each of the at least two openings are covered by a separate barrier.

16. A method of preparing an aseptic package (10) containing a medical device (30) or delivery system according to any one of claims 1-15, comprising:
placing the medical device or delivery system in the cavity (16) of the package;
sealing the first member or section to the second member or section at the peripheral seal (22);
at least one of vacuum drying, nitrogen purging, heat drying, and terminal sterilization of an interior of the package; and
sealing the first member or section to the second member or section at the sealable member (24) such that the cavity (16) is no longer in communication with the at least one opening (18) such that the sealed package is hermetically sealed, the at least one opening (18) being in the package after the cavity is no longer in communication with the at least one opening.

17. The method of claim 16, comprising at least two of vacuum drying, nitrogen purging, heat drying, and terminal sterilization of an interior of the package prior to sealing the first member (12) or first section (68) to the second member (14) or second section (70) at the sealable member.

18. The method of any one of claims 16 or 17, further comprising:
sterilizing the package after the first sealing step,
preferably wherein the sterilizing step is selected from at least one of ethylene oxide sterilization, gamma electron-beam sterilization, and low temperature oxidative sterilization, or
wherein the sterilizing step includes a combination of two or more of ethylene oxide sterilization, gamma electron-beam sterilization, and low temperature oxidative sterilization.

## Patentansprüche

1. Gehäuse (10) für eine medizinische Vorrichtung (30) oder ein Lieferungssystem, Folgendes umfassend:
ein erstes Bauteil (12), wobei das erste Bauteil im Wesentlichen planar ist;
ein zweites Bauteil (14), das mindestens einen Hohlraum (16) zum Aufnehmen einer medizinischen Vorrichtung (30) oder eines Lieferungssystems umfasst, wobei das erste und das zweite Bauteil derartig gegenüberliegen, dass das erste Bauteil mindestens den Hohlraum des zweiten planaren Bauteils abdeckt;
eine periphere Dichtung (22), die zwischen dem ersten Bauteil und dem zweiten Bauteil zum Versiegeln des ersten Bauteils mit dem zweiten Bauteil an dem Äußeren von einem oder von beiden des ersten Bauteils und des zweiten Bauteils positioniert ist
mindestens eine Öffnung (18), die in mindestens einem des ersten und des zweiten Bauteils ausgebildet ist;
eine mikrobenbeständige Sperre (20), welche die mindestens eine Öffnung (18) abdeckt;
**dadurch gekennzeichnet, dass**
ein versiegelbares Bauteil (24), das zwischen dem ersten Bauteil und dem zweiten Bauteil positioniert ist, welches das erste und das zweite Bauteil an einer Position zwischen der mindestens einen Öffnung und dem Hohlraum derartig zusammenklebt, dass, wenn das erste und das zweite Bauteil durch das versiegelbare Bauteil verklebt werden, der Hohlraum nicht länger in Verbindung mit der mindestens einen Öffnung ist, wobei die mindestens eine Öffnung (18), die durch die mikrobenbeständige Sperre (20) abgedeckt ist, in dem Gehäuse (10) vorhanden ist, nachdem der Hohlraum nicht länger in Verbindung mit der mindestens einen Öffnung ist.

2. Gehäuse (10) für eine medizinische Vorrichtung (30) oder ein Lieferungssystem, Folgendes umfassend:
ein Bauteil (66) mit einem ersten Abschnitt (68), der im Wesentlichen planar ist, und mit einem zweiten Abschnitt (70), der dem ersten Abschnitt benachbart ist;
mindestens einen Hohlraum (16) zum Aufnehmen einer medizinischen Vorrichtung oder eines Lieferungssystems, das in dem zweiten Abschnitt positioniert ist, wobei das Bauteil (66) entlang einer Achse (72) zwischen dem ersten und dem zweiten Abschnitt derartig faltbar ist, dass der erste und der zweite Abschnitt gegenüberliegen und der erste Abschnitt mindestens den Hohlraum des zweiten Abschnitts abdeckt, wenn die Abschnitte gegenüberliegen;
eine periphere Dichtung (22), die auf mindestens einem des ersten oder des zweiten Abschnitts zum Versiegeln des ersten Abschnitts mit dem zweiten Abschnitt an dem Äußeren von einem oder von beiden des ersten Abschnitts und des zweiten Abschnitts positioniert ist, wenn die Abschnitte gegenüberliegen;
mindestens eine Öffnung (18), die in mindestens einem des ersten und des zweiten Abschnitts ausgebildet ist;
eine mikrobenbeständige Sperre (20), welche die mindestens eine Öffnung abdeckt; und
ein versiegelbares Bauteil (24), welches den ersten und den zweiten Abschnitt an einer Position zwischen der mindestens einen Öffnung und dem Hohlraum derartig zusammenklebt, dass, wenn der erste und der zweite Abschnitt durch das versiegelbare Bauteil verklebt werden, der Hohlraum nicht länger in Verbindung mit der mindestens einen Öffnung ist, wobei die mindestens eine Öffnung (10), die durch die mikrobenbeständige Sperre (20) abgedeckt wird, in dem Gehäuse (10) vorhanden ist, nachdem der Hohlraum nicht länger in Verbindung mit der mindestens einen Öffnung ist.

3. Gehäuse nach Anspruch 1 oder 2, wobei die Sperre (20) aus einem antimikrobischen Material oder einem mikrobenundurchlässigen Material ausgebildet ist,
wobei vorzugsweise das antimikrobische Material oder das mikrobenundurchlässige Material Polyolefinfasern umfasst und
wobei besonders bevorzugt das Polyolefin aus Polyethylen oder Polypropylen ausgewählt ist und wobei die Polyolefinfasern nicht gewebt sind.

4. Gehäuse nach einem vorhergehenden Anspruch, wobei die mindestens eine Öffnung (18) in dem ersten Bauteil (12) oder in dem ersten Abschnitt (68) ausgebildet ist und die Sperre (20) an dem ersten Bauteil oder an dem ersten Abschnitt angebracht ist, der die Gesamtheit der mindestens einen Öffnung abdeckt, oder
wobei die mindestens eine Öffnung (18) in dem zweiten Bauteil (14) oder in dem zweiten Abschnitt (70) ausgebildet ist und die Sperre an dem zweiten Bauteil oder an dem zweiten Abschnitt angebracht ist, der die Gesamtheit der mindestens einen Öffnung abdeckt.

5. Gehäuse nach Anspruch 4, weiterhin eine Sperrendichtung (26), die zwischen dem ersten Bauteil (12) oder dem ersten Abschnitt (68) und der Sperre positioniert ist, um die Sperre an dem ersten Bauteil oder an dem ersten Abschnitt zu befestigen, oder eine Sperrendichtung (26) umfassend, die zwischen dem zweiten Bauteil (14) oder dem zweiten Abschnitt (70) und der Sperre positioniert ist, um die Sperre an dem zweiten Bauteil oder an dem zweiten Abschnitt zu befestigen.

6. Gehäuse nach einem vorhergehenden Anspruch, wobei die Sperre (20) eine atmungsaktive Sperre ist.

7. Gehäuse nach einem vorhergehenden Anspruch, wobei mindestens eines des ersten Bauteils (12) oder des ersten Abschnitts (68) oder des zweiten Bauteils (14) oder des zweiten Abschnitts (70) aus einem Material ausgebildet ist, das aus einem gasundurchlässigen Polymer und einer Metallfolie ausgewählt ist,
wobei vorzugsweise jedes des ersten und des zweiten Bauteils oder Abschnitts aus einer Metallfolie ausgebildet ist und wobei besonders bevorzugt die Metallfolie aus einer Aluminiumfolie und einer Edelstahlfolie ausgewählt ist.

8. Gehäuse nach einem vorhergehenden Anspruch, wobei mindestens ein Element von der peripheren Dichtung (22), dem versiegelbaren Bauteil (24) oder der Sperrendichtung (26) aus einer Klebstoffbeschichtung ausgebildet ist, wobei vorzugsweise die Klebstoffbeschichtung aus einer heißversiegelbaren Klebstoffbeschichtung und einer druckversiegelbaren Klebstoffbeschichtung ausgewählt ist.

9. Gehäuse nach einem vorhergehenden Anspruch, weiterhin ein Trockenmittel umfassend.

10. Gehäuse nach einem vorhergehenden Anspruch, wobei das Innere des Gehäuses steril ist, wenn das erste Bauteil (12) oder der erste Abschnitt (68) und das zweite Bauteil (14) oder der zweite Abschnitt (70) miteinander versiegelt werden.

11. Gehäuse nach einem vorhergehenden Anspruch, wobei der Hohlraum (16) eine Gestalt aufweist, die aus rechteckig, quadratisch, polygonal, oval oder kreisförmig ausgewählt ist.

12. Gehäuse nach einem vorhergehenden Anspruch, wobei das erste Bauteil (12) oder der erste Abschnitt (68) mindestens über einen Teil des zweiten Bauteils (14) oder des zweiten Abschnitts (70) an einem Rand übersteht, wenn das erste und das zweite Bauteil oder die Abschnitte miteinander versiegelt werden.

13. Gehäuse nach einem vorhergehenden Anspruch, wobei das erste Bauteil (12) oder der erste Abschnitt (68) und das zweite Bauteil (14) oder der zweite Abschnitt (70) jeweils einen Außenrand aufweisen, wobei die Außenränder im Wesentlichen aneinander ausgerichtet sind, wenn das erste und das zweite Bauteil oder die Abschnitte miteinander versiegelt werden.

14. Gehäuse nach einem vorhergehenden Anspruch, wobei das versiegelbare Bauteil (24) die periphere Dichtung (22) an zwei Seiten des Äußeren des ersten und des zweiten Bauteils oder der Abschnitte schneidet.

15. Gehäuse nach einem vorhergehenden Anspruch, wobei die mindestens eine Öffnung (18) mindestens zwei Öffnungen umfasst, wobei jede der mindestens zwei Öffnungen durch eine einzige Sperre abgedeckt ist oder wobei jede der mindestens zwei Öffnungen durch eine separate Sperre abgedeckt ist.

16. Verfahren zum Zubereiten eines aseptischen Gehäuses (10), das eine medizinische Vorrichtung (30) oder ein Lieferungssystem nach einem der Ansprüche 1 bis 15 enthält, Folgendes umfassend:
Anordnen der medizinischen Vorrichtung oder des Lieferungssystems in dem Hohlraum (16) des Gehäuses;
Versiegeln des ersten Bauteils oder Abschnitts mit dem zweiten Bauteil oder Abschnitt an der peripheren Dichtung (22);
mindestens eines aus Vakuumtrocknen, Stickstoffspülen, Hitzetrocknen und Endsterilisation eines Inneren des Gehäuses; und
Versiegeln des ersten Bauteils oder Abschnitts mit dem zweiten Bauteil oder Abschnitt an dem versiegelbaren Bauteil (24), so dass der Hohlraum (16) nicht länger in Verbindung mit der mindestens einen Öffnung (18) ist, so dass das versiegelte Gehäuse hermetisch versiegelt ist, wobei die mindestens eine Öffnung (18) in dem Gehäuse ist, nachdem der Hohlraum nicht länger in Verbindung mit der mindestens einen Öffnung ist.

17. Verfahren nach Anspruch 16, mindestens zwei Schritte aus Vakuumtrocknen, Stickstoffspülen, Hitzetrocknen und Endsterilisation eines Inneren des Gehäuses vor dem Versiegeln des ersten Bauteils (12) oder des ersten Abschnitts (68) mit dem zweiten Bauteil (14) oder dem zweiten Abschnitt (70) an dem versiegelbaren Bauteil umfassend.

18. Verfahren nach einem der Ansprüche 16 oder 17, weiterhin Folgendes umfassend:
Sterilisieren des Gehäuses nach dem ersten Versiegelungsschritt,
wobei vorzugsweise der Schritt des Sterilisierens aus mindestens einem aus Ethylenoxidsterilisation, Gamma-Elektronenstrahlsterilisation und Tieftemperatur-Oxidationssterilisation ausgewählt ist oder
wobei der Schritt des Sterilisierens eine Kombination aus zwei oder mehr Schritten aus Ethylenoxidsterilisation, Gamma-Elektronenstrahlsterilisation und Tieftemperatur-Oxidationssterilisation umfasst.

## Revendications

1. Emballage (10) pour un dispositif médical (30) ou un système de délivrance, comprenant :
un premier élément (12), le premier élément étant sensiblement plan ;
un deuxième élément (14) comprenant au moins une cavité (16) destinée à recevoir un dispositif médical (30) ou un système de délivrance, les premier et deuxième éléments se faisant face de telle sorte que le premier élément recouvre au moins la cavité du deuxième élément plan ;
un joint d'étanchéité périphérique (22) positionné entre le premier élément et le deuxième élément pour sceller le premier élément sur le deuxième élément à la périphérie du premier élément ou du deuxième élément ou des deux ;
au moins une ouverture (18) formée dans au moins un des premier et deuxième éléments ;
une barrière résistante aux microbes (20) recouvrant l'au moins une ouverture (18) ;
**caractérisé par**
un élément scellable (24) positionné entre le premier élément et le deuxième élément, qui colle les premier et deuxième éléments ensemble à une position entre l'au moins une ouverture et la cavité de telle sorte que, quand les premier et deuxième éléments sont collés par l'élément scellable, la cavité n'est plus en communication avec l'au moins une ouverture, l'au moins une ouverture (18) recouverte par la barrière résistante aux microbes (20) étant présente dans l'emballage (10) une fois que la cavité n'est plus en communication avec l'au moins une ouverture.

2. Emballage (10) pour un dispositif médical (30) ou un système de délivrance, comprenant :
un élément (66) ayant une première section (68) qui est sensiblement plane et une deuxième section (70) adjacente à la première section ;
au moins une cavité (16) destinée à recevoir un dispositif médical ou un système de délivrance positionnée dans la deuxième section, l'élément (66) étant pliable le long d'un axe (72) entre les première et deuxième sections de telle sorte que les première et deuxième sections se font face et la première section recouvre au moins la cavité de la deuxième section quand les sections se font face ;
un joint d'étanchéité périphérique (22) positionné sur au moins une des première et deuxième sections pour sceller la première section sur la deuxième section à la périphérie de la première section ou de la deuxième section ou des deux quand les sections se font face ;
au moins une ouverture (18) formée dans au moins une des première et deuxième sections ;
une barrière résistante aux microbes (20) recouvrant l'au moins une ouverture ; et
un élément scellable (24) qui colle les première et deuxième sections ensemble à une position entre l'au moins une ouverture et la cavité de telle sorte que, quand les première et deuxième sections sont collées par l'élément scellable, la cavité n'est plus en communication avec l'au moins une ouverture, l'au moins une ouverture (10) recouverte par la barrière résistante aux microbes (20) étant présente dans l'emballage (10) une fois que la cavité n'est plus en communication avec l'au moins une ouverture.

3. Emballage de la revendication 1 ou 2, dans lequel la barrière (20) est constituée d'un matériau antimicrobien ou d'un matériau imperméable aux microbes,
de préférence dans lequel le matériau antimicrobien ou matériau imperméable aux microbes est composé de fibres de polyoléfine, et
mieux encore dans lequel la polyoléfine est choisie parmi le polyéthylène et le polypropylène, et dans lequel les fibres de polyoléfine ne sont pas tissées.

4. Emballage d'une quelconque revendication précédente, dans lequel l'au moins une ouverture (18) est formée dans le premier élément (12) ou la première section (68) et la barrière (20) est attachée au premier élément ou à la première section recouvrant la totalité de l'au moins une ouverture, ou
dans lequel l'au moins une ouverture (18) est formée dans le deuxième élément (14) ou la deuxième section (70) et la barrière est attachée au deuxième élément ou à la deuxième section recouvrant la totalité de l'au moins une ouverture.

5. Emballage de la revendication 4, comprenant en outre un joint d'étanchéité de barrière (26) positionné entre le premier élément (12) ou la première section (68) et la barrière pour fixer la barrière au premier élément ou à la première section, ou un joint d'étanchéité de barrière (26) positionné entre le deuxième élément (14) ou la deuxième section (70) et la barrière pour fixer la barrière au deuxième élément ou à la deuxième section.

6. Emballage d'une quelconque revendication précédente, dans lequel la barrière (20) est une barrière respirante.

7. Emballage d'une quelconque revendication précédente, dans lequel l'un au moins du premier élément (12) ou de la première section (68) et du deuxième élément (14) ou de la deuxième section (70) est constitué d'un matériau choisi parmi un polymère perméable aux gaz et une feuille métallique,
de préférence dans lequel chacun des premier et deuxième éléments ou sections est constitué d'une feuille métallique, et mieux encore dans lequel la feuille métallique est choisie parmi une feuille d'aluminium et une feuille d'acier inoxydable.

8. Emballage d'une quelconque revendication précédente, dans lequel l'un au moins du joint d'étanchéité périphérique (22), de l'élément scellable (24) et du joint d'étanchéité de barrière (26) est constitué d'un revêtement adhésif,
de préférence dans lequel le revêtement adhésif est choisi parmi un revêtement adhésif thermoscellable et un revêtement adhésif scellable sous pression.

9. Emballage d'une quelconque revendication précédente, comportant en outre un absorbeur d'humidité.

10. Emballage d'une quelconque revendication précédente, l'intérieur de l'emballage étant stérile quand le premier élément (12) ou la première section (68) et le deuxième élément (14) ou la deuxième section (70) sont scellés ensemble.

11. Emballage d'une quelconque revendication précédente, dans lequel la cavité (16) a une forme choisie parmi rectangulaire, carrée, polygonale, ovale, et circulaire.

12. Emballage d'une quelconque revendication précédente, dans lequel le premier élément (12) ou la première section (68) est en surplomb d'au moins une portion du deuxième élément (14) ou de la deuxième section (70) au niveau d'un bord quand les premier et deuxième éléments ou sections sont scellés ensemble.

13. Emballage d'une quelconque revendication précédente, dans lequel le premier élément (12) ou la première section (68) et le deuxième élément (14) ou la deuxième section (70) ont chacun une périphérie extérieure, les périphéries extérieures étant sensiblement alignées quand les premier et deuxième éléments ou sections sont scellés ensemble.

14. Emballage d'une quelconque revendication précédente, dans lequel l'élément scellable (24) croise le joint d'étanchéité périphérique (22) sur deux côtés de la périphérie des premier et deuxième éléments ou sections.

15. Emballage d'une quelconque revendication précédente, dans lequel l'au moins une ouverture (18) comporte au moins deux ouvertures, dans lequel chacune des au moins deux ouvertures est recouverte par une seule barrière ou dans lequel chacune des au moins deux ouvertures est recouverte par une barrière distincte.

16. Procédé de préparation d'un emballage aseptique (10) contenant un dispositif médical (30) ou un système de délivrance selon l'une quelconque des revendications 1 à 15, comprenant :
le positionnement du dispositif médical ou du système de délivrance dans la cavité (16) de l'emballage ;
le scellement du premier élément ou section sur le deuxième élément ou section au niveau du joint d'étanchéité périphérique (22) ;
au moins une opération parmi le séchage sous vide, la purge à l'azote, le séchage thermique et la stérilisation terminale d'un intérieur de l'emballage ; et
le scellement du premier élément ou section sur le deuxième élément ou section au niveau de l'élément scellable (24) de telle sorte que la cavité (16) n'est plus en communication avec l'au moins une ouverture (18) de telle sorte que l'emballage scellé est scellé hermétiquement, l'au moins une ouverture (18) étant dans l'emballage une fois que la cavité n'est plus en communication avec l'au moins une ouverture.

17. Procédé de la revendication 16, comprenant au moins deux opérations parmi le séchage sous vide, la purge à l'azote, le séchage thermique et la stérilisation terminale d'un intérieur de l'emballage avant le scellement du premier élément (12) ou de la première section (68) sur le deuxième élément (14) ou la deuxième section (70) au niveau de l'élément scellable.

18. Procédé de l'une quelconque des revendications 16 et 17, comprenant en outre :
la stérilisation de l'emballage après la première étape de scellement,
de préférence dans lequel l'étape de stérilisation est choisie à partir d'au moins une opération parmi une stérilisation à l'oxyde d'éthylène, une stérilisation gamma/par faisceau d'électrons, et une stérilisation oxydative à basse température, ou
dans lequel l'étape de stérilisation comporte une combinaison d'au moins deux opérations parmi une stérilisation à l'oxyde d'éthylène, une stérilisation gamma/par faisceau d'électrons, et une stérilisation oxydative à basse température.
